# EUROPEAN PATENT SPECIFICATION

(11) **EP 1 028 692 B1**
(45) Date of publication and mention of the grant of the patent: **26.03.2003**
(21) Application number: 98950269.5
(22) Date of filing: 12.11.1998
(51) Int. Cl.: A61K 6/02, A61K 6/08, A61K 6/00

(54) **METAL-FREE DENTAL FILLING SYSTEM AS A SUBSTITUTE FOR AMALGAM**
METALLFREIES ZAHNFÜLLSYSTEM ALS ERSATZ FÜR AMALGAM
SYSTEME D'OBTURATION DENTAIRE NE CONTENANT PAS DE METAUX, UTILISE EN TANT QUE PRODUIT DE SUBSTITUTION DE L'AMALGAME

(30) Priority: 13.11.1997 DE 19750319; 21.11.1997 US 66128 P
(43) Date of publication of application: 23.08.2000
(73) Proprietor: 3M ESPE AG, 82229 Seefeld (DE); Krejci, Daniela, 1296 Coppet (CH)
(72) Inventor: KREJCI, Daniela, CH-1296 Coppet (CH); KREJCI, Ivo, CH-1296 Coppet (CH); LUTZ, Felix, CH-8706 Meilen (CH)
(86) International application number: IB9801798
(87) International publication number: WO99025309

(56) References cited:
- EP-A- 0 423 430
- DE-A- 3 634 697
- DE-A- 4 445 266
- DE-A- 19 544 670
- DE-A- 19 603 577
- US-A- 4 425 094
- US-A- 4 685 969
- US-A- 4 872 936
- DATABASE WPI Section Ch, Week 8825 Derwent Publications Ltd., London, GB; Class A96, AN 88-275902 XP002103573 & JP 63 203 604 A (TOKUYAMA SODA KK) , 23 August 1988

## Description

### Cross References to Related Applications

This application claims the priority of the German patent application 197 50 319.5, filed on November 13, 1997, and U.S. provisional application 60/066,128 filed on November 21, 1997.

### Technical Field

The present invention relates to a metal-free dental filling system for filling therapy or root filling therapy, respectively, as well as to its use as a substitute for amalgam.

### Background Art

For tooth restoration in dentistry, three hierarchically ordered goals are pursued, whereby each higher standard is based on the fulfillment of the lower standard or the lower standards.
- The partial goal of the lowest standard 1 consists in the conservation of the dental hard tissue and the protection of the pulpa.
- Standard 2 is further aiming at restoring the tooth's shape and function.
- The purpose of standard 3 is to design the restoration in such a way that it is imperceptible at conversation distance and remain so throughout the stipulated service life.

Amalgams, i.e. mixtures of a silver-tin alloy with liquid mercury, have been used for more than 150 years as standard filling material, in particular for posterior, permanent teeth. When used with adequate operative techniques, amalgam restorations are able to protect the dental hard tissue for years and to restore the tooth's shape as well it's functionality. Thus, amalgam restorations fully satisfy standard 2. In filling therapy with amalgam, a comparatively simple operative technique has evolved that relies on steel matrices and wooden wedges for giving the filling its correct, preferably overhang-free contour at its outer surfaces. Occlusal shaping is carried out by carving. Amalgam fillings are furthermore comparatively cheap. The medium survival rate of amalgam fillings can be 10 years and more.

In recent years, however, amalgams-have lost quite some ground as a standard restorative material. The main reasons of this decline are, among others, its controversial toxic and allergenic potential, its environmental impact and its lack of tooth color. As a consequence, it was necessary to search for materials or material systems that could be used to replace amalgam for posterior teeth, in particular for permanent teeth and stress-bearing restorations. This resulted in two material or system groups differing in their purpose, namely amalgam alternatives and amalgam substitutes.

Amalgam alternatives have to suffice standard 3 and not only have to guarantee the conservation of the dental hard tissue as well as shape and function of the restored tooth over a long time, but they also must be and remain imperceptible at normal conversation distance. This group of materials in particular comprises composite fillings and composite or ceramic work-pieces. This not only requires tooth color, but also a stress resistant, perfect marginal adaptation. With fillings, the latter is achieved by using a rather complex operative technique, with work-pieces by high accuracy in conjunction with efficient adhesive or combined adhesive/luting systems.

In contrast to this, an amalgam substitute has to satisfy standard 2 just as amalgam does; in addition to this, the operative technique should be simple for cost reasons and be as close as possible to the known amalgam technique, i.e. it should be possible to work with steel matrices and wooden wedges, to use a simple incremental technique and, if light curing materials are used, irradiation from an occlusal direction should suffice.

Various amalgam substitute systems are known:

DE-196 03 577 A1 relates to an adhesive system, wherein a gap free connection between the plastic filling material and the dental hard tissue is aimed at. In such a system, the bond between dental hard tissue and filling material is excellent, such that in case of adhesive failures, continuity fractures occur in the dental hard tissue or in the restorative material. This means that no caries protection is provided along these marginal openings if they run in the dental hard tissue.

DE 195 44 670 describes an adhesive system that does not harden by radical polymerization but rather as a product of condensation. In this way, an oxygen-inhibited surface layer is to be avoided. Thus, however, the adhesive may be better suited than usual, radically polymerizing sealants for sealing caries. In the application as an adhesive, where, according to the inventor, the prevention of margin gap formation is again aimed at, cohesive failure results in the dentin and in the enamel. Hence, this system is unable to reliably protect the dental hard tissue. With comparatively frequent adhesive failure, the enamel is not protected at all in the area of the marginal openings, which are therefore not desired at all. In dentin, a partial, erratic, unpredictable protection is to be expected where the bond happens to break between the adhesive and the filling material and adhesive islands remain on the dental hard tissue. The described system therefore provides no safe and complete protection of the enamel or dentin, and it also belongs to the category of the adhesive systems aiming at a total bond between the dental hard tissue and the restorative material.

EP 0 423 430 describes a dentin-adhesive system using a primer and a bond on dentin. Again, it is aimed at a total leakage-free bond between dental hard tissue and restorative material.

EP 0 088 527 describes an enamel conditioner designed to generate a better bond between the enamel and the restorative material than it used to be the case upon the usual etching process by means of phosphoric acid.

DE 34 14 163 relates on a dentin primer and describes a dentin bonding system working with a primer and a bond on dentin, wherein again a total leakage-free bond between dental hard tissue and restorative material is desired.

In US 4,685,969 a process for filling a tooth cavity with a composite dental filling is disclosed, the process comprising i.a. the step of introducing a separating substane into a cavity to form a separating layer in said cavity, said separating substance decomposing at body temperature into a sol. The separating substance facilitates the removal of a composite dental filling such as an inlay from the cavity.

US 4,425,094 relates to a method of filling a tooth in root canal therapy, the filling system comprising a solid shaped body swellable when placed into a root canal comprising hydrophilic, polymeric compositions.

US 4,872,936 refers to a polymerizable cement mixture containing (a) polymerizable unsaturated monomers and/or oligomers and/or prepolymers containing acid groups, (b) reactive fillers and (c) curing agents. The mixture can be cured both radically and via ionic reaction.

None of the adhesive systems according to the state of the art, however, uses enamel and dentin bonding selectively for protection and adhesive sealing of the dental hard tissue, while selectively renouncing on the requirement of a bond between the shrinking restorative material and the sealed dental hard tissue.

The adhesive systems according to the state of the art are fully unsatisfactory when being used in combination with amalgam substitutes and the corresponding operative techniques, because unpredictably located fractures in the restorative material, the interface bet-ween restorative material and adhesive, within the adhesive, at the interface between the adhesive and the dental hard tissue and/or within the dental hard tissue can occur. Fractures at the interface between the adhesive and the dental hard tissue and within the dental hard tissue are particularly damaging. A reliable protection cannot be achieved using the required simple placement techniques and currently available adhesive systems.

Investigations have shown that fillings made of an amalgam substitute according to the state of the art in combination with the pertinent adhesive system, such as composite-adhesive systems, or compomer-adhesive systems (i.e. polyacid-modified composites) and composite-adhesive systems specifically developed as amalgam substitutes, result in fillings which, when occlusally loaded, show after a short period of time, possibly within months, partially even initially, over 60 %, partially even up to 95 % and more of the total margin length, open margins, quite often wide open margins. This certainly results in marginal discoloration, which might still be acceptable within the requirements of operative standard 2, but certainly do result in inaccuracies regarding the medium-range caries diagnostic. The risk for secondary caries with fillings of the known material combinations mentioned above, must therefore be evaluated being high to quite high in view of the known, clinical experience and the available in-vitro potential assessments.

The materials presently used as amalgam substitutes therefore do not meet even operative standard 1, i.e. tooth conservation can not or hardly be achieved over a medium time range. Such materials may therefore only be used as temporary fillings.

The reason for the failure of the amalgam substitute materials according to the state of the art lies in the fact that such materials usually shrink by 2.5 to 4.5 volume percent during the curing process. The resulting stress build-up damages the bonds as generated by the adhesive systems according to the state of the art and exceeds the cohesive strength of the dental hard tissue, especially in the enamel, and of the filling material itself. Under stress, non-predictable continuity fractures occur, namely fissures in the enamel and, more rarely, in the dentin, a breaking of the bond between dental hard tissue and adhesive system, within the adhesive system, at the interface between adhesive system and filling, and within the filling. Even though an adhesive system is used, unprotected, adhesive free areas of dental hard tissue are exposed, either due to cohesive fractures within the dental hard tissue or due to a breaking of the bond between the adhesive system and the dental hard tissue.

### Disclosure of the Invention

Hence, it is a general object of the invention to provide a dental filling system that avoids at least part of the above disadvantages and problems.

In particular, it was an aim of the present invention to provide an amalgam substitute, which fulfills the above partial goals 1 and 2 as well as, possibly, 3.

This object is achieved according to claim 1. Preferred embodiments are described in the dependent claims.

The metal free, dental filling system for the filling therapy of cavities or for the filling therapy of root canals according to the present invention consists of or comprises
a) a dental filling material for cavities or root canals, as well as
b) an adhesive, curing or self-curing sealant for the dental hard tissue,
c) wherein the adhesive bond of the cured adhesive sealant to the dental hard tissue, i.e. the enamel, dentin and root cement, is substantially stronger than to the cured filling material such that substantially no destructive forces are transmitted from the shrinking filling material to the adhesive sealant.

In an especially preferred embodiment the filling system according to the invention comprises an intermediate layer material d), which is suitable to be arranged between the dental filling material a) and the sealant b), wherein the adhesive bond between the cured adhesive sealant b) to the dental hard tissue is much stronger than to the intermediate layer material d), such that substantially no destructive forces are transmitted from the shrinking filling material to the adhesive sealant.

Preferably, said intermediate layer material d) is removable before application of the filling material.

In an especially preferred embodiment, substantially no adhesive bond is formed between the cured sealant b) to the filling material a) or, if applicable, to the intermediate layer d), respectively, such that during the curing no destructive forces at all are transmitted from the shrinking filling material a) and/or the intermediate layer material d) to the adhesive sealant b).

Hence, in contrast to the known state of the art the present invention does not aim at protecting the dental hard tissue by a perfect bond to the filling material. The present invention rather provides an isolating, selective adhesive seal of the dental hard tissue, thereby generating the desired protection in the area of the margin gaps.

Preferably, the desired separation or, compared to the destructive stress build-up within the filling, the weak, no stress transferring bonds between the sealant and the filling material are achieved by making known adhesive coatings unable to co-polymerize or lock by a suitable choice of monomers, admixtures or curing methods. Alternatively, instead of using a known adhesive system, an adhesive system is used that adheres to the enamel and dentin, forms a coating, is chemically and physically resistant but does not form a mechanical or chemical bond to the filling system that might endanger its own bond to the hard dental material.

Using the system according to the invention, the dental hard tissue remains free of fractures and is protected against caries and erosive processes throughout the stipulated service life of the filling. The amalgam substitute according to the invention therefore meets standard 2 by a complete prevention of the risk of secondary caries and by preserving the dental hard tissue, while shape and function of the tooth are restored by the filling material.

### Modes for Carrying Out the Invention

The main object of the present invention is a dental filling system that renounces on a total bond from the sealant b) for the dental hard tissue to the filling material a) or the intermediate layer material d), respectively. An isolated, selective adhesive sealing of the dental hard tissue can be reached as follows:
A) A transmission of shrinking-forces can be avoided by preventing mechanical interlocking and homo-/co-polymerization between the dental filing material a) and, if present, the intermediate layer material d) on the one hand, as well as the adhesive sealant b) for the dental hard tissue on the other hand. This can e.g. be achieved by entirely coating the sealant b) by a separation layer. For this purpose, painting materials, such as zinc oxide-Eugenol, said compound being known to the man of the art, barrier materials, such as powders or powder coatings, or glycerol-acetate-based isolating gels, or primers, e.g. silicon-organic compounds.
B) The undesired connection with the filling material can further be reached by a reduction of the free radicals at the surface of the radically polymerizing sealant. In a further preferred embodiment, the undesired transmission of shrinking-forces is therefore prevented by the fact the hardened adhesive sealant b) has a low free radical content and is therefore not apt for co-polymerization.
   It is especially preferred that the hardened sealant b) is even unable to undergo homo- and/or co-polymerization. The ability for co-polymerization of the components a), b) and d) can e.g. affected and weakened by admixing of BisGMA bicarbonate (of the Bayer Company) or photo-initiators in higher concentrations, or by coating the sealant b) with an arbitrary air blocker as known to the person skilled in the art before light curing for preventing the build-up of an oxygen-inhibited surface layer.
   On the other hand, a reduction of the free radicals at the surface of a radically polymerizing sealant can also be reached by a suitable choice of the monomer systems.
C) In a further preferred embodiment a transmission of shrinking-forces is reduced or diminished by using filling materials a) or, if present, intermediate layer materials d) and adhesive sealants b) of different polarity, notably for suppressing wetting. It is especially preferred to use a hydrophilic or amphiphilic sealant for the dental hard tissue and a strongly hydrophobic filling system. Particularly preferred is thereby a sealant b) being hydrophilic or amphiphilic, whereby the filling material a) is strongly hydrophobic, e.g. Etch & Prime 3.0 or Prime & Bond NT (of the Dentply company) combined with Adaptic II (Johnson & Johnson)or Pertac (Espe company). Also preferred is a sealant b) being hydrophobic whereas the filling material is strongly hydrophilic, e.g. Visiobond (Espe company)in combination with PhotacFil (Espe company) or Ketac (Espe company) Bond.
D) Especially, if the dental filling material a) is a methacrylated substance, the adhesive sealant b) has a separating effect in respect to the filling material a) due to a different polymerization that does not allow a co- or homo-polymerisation of di- or polyacrylatesystems. This prevents a shrinking-force transmitting bond between the adhesive sealant and the filling material. Preferred sealants for the dental hard tissue are selected from the group comprising water glass, silica ester hydrolysates, polymerizable silane compounds, natural resin varnishes, cyanate based varnishes, epoxide resins, epoxidated soy oil or epoxy-modified silicones. They can preferably be selected as follows:
   - water glass: e.g. a water glass glue in colloidal solution of potassium or sodium silicate, wherein the setting takes place, e.g. through evaporation of the water share.
   - silica ester hydrolysates: Through the preparation of partial hydrolisates of silica ester hydrolysates, solid resin coatings can be achieved. The mode of preparation of such a coating could be e.g. vinyltriethoxysilane 12 % per weight, silica ester TES (Dynamit Nobel) 34 % per weight, phosphoric acid 0,3 % per weight dissolved in acetone. After the evaporation of the solvent, clear, hard, shining hydrophobic films are formed. The adhesion to substrates such as dentin and enamel can be adjusted by means of metal acetylacetonates.
   - polymerizable silane compounds: preferably silanes being modified with epoxide- or metacrylate groups. The mentioned reactive silane monomers could contain metal complexes having an affinity to the dentin or the enamel, e.g. compounds with calcium, fluorine or other halides.
   - natural resin varnishes : Through hydrophobic natural resin varnishes forming films, dense, hydrophobic coatings could be achieved, e.g. following the preparations of Colophonium 12 % per weight, Schellack 25 % per weight, Gummi arabicum 8 % per weight, Cellulose 5 % per weight and Alkohol DAB ad 100.
   - epoxy resins : As an example the epoxide-modified vinylester resins, polyester resins, vinylether-resins oder bisphenol-A-epoxid resin (Dow Corning) Deracane Oligomere; vectomer-vinylether oligomeres, ISP-vinylether (ISP company), CIBA Araldit types (Ciba-Geigy company); Union Carbide cycloaliphatic epoxides are to be mentioned. Depending on their modification, said raw materials could be polymerized through radical or ionic initiation, thus providing 2-component systems hardening chemically, e.g. following to the preparation comprising the component A : bisphenol-A-epoxy resin (D.E.R. 335 Dow Corning); Cumene hydroperoxide and component B: bisphenol-A-epoxyresin, silane Z-6020 (Dow Corning) and triethanolamine. Correspondingly the formulations that harden upon light exposure are conceivable with light in the visible or ultra-violet range, e.g. following to the preparation Vectomer 2010 urethan oligomer (of the Vectomer company) 45 % per weight, monomer 4010 15 % per weight, Rapicure CHVE (of the ISP company) monomer 39 % per weight (1,4-cyclohexane-dimethanole-divinylether), cationic initiator UVI-6974 1 % per weight.
   - epoxidated soy oils : They are cross-linkable through cationic or radical initiation, e.g. Henkel Edenol types; further examples for reactive oligomers are cyracure oligomeres (Union Carbide), cycloaliphatic epoxide resins like UVR 61110 (Dow Corning), i.e. 3,4-epoxycyclohexylmethyl-3,4-epoxycyclohexane-carboxylate, or UVR 6128 (Dow Corning), i.e. bis-(3,4 epoxycyclohexyl-adipate or UVR 6216 (Dow Corning), i.e. 1,2-epoxyhexadecane.
   - methacrylic or epoxy-modified silicones (e.g. of Goldschmidt, Wacker, Mitsubishi) as well as methacryl-modified gelatines and cellulose esters also display good film forming properties : they could also serve as film-forming carrier materials for a variety of active agent, like fluorides or antiseptic or bactericidal additives. The desired separation effect is notably achieved by employing 2 different synthesis forms. Said process could be achieved through different cross-linkage mechanisms, like the chemical hardening, the light-induced hardening, the radical-or cationic induced hardening, cross-linking through radical condensation etc.

The filling materials a) comprise plastics, composites, compomers, ormoceres, ceromeres, polyglasses, glass ionomer cements, carboxylate cement, phosphate cement, EBA-cement (Espe company) or guttapercha, or consist thereof. These materials are preferably photo curing, chemically curing, dually curing, heat curing or thermoplastic.

The above mentioned compounds are commercially available and are generally known by persons skilled in the art.

The sealants b) for the dental hard tissue exhibit a good bond with the dental hard tissues. They form a leakage-free and gap-free, closed, mechanically and chemically resistive protective layer and, if possible, release (as a "smart material") caries protective agents, i.e. they release plaque-suppressing or caries-protective agents permanently or upon request, such as flourides, chlorhexidine, triclosan, calcium or hydroxyl-ions. Preferably used sealants for the dental hard tissue are photo hardening, chemically hardening, dually curing, heat curing or thermoplastic or they cure using the principles of contact adhesives.

In a highly preferred embodiment the dental filling material a) is photo hardening (i.e. hardening upon exposure to light of a suitable wave-length) and the sealant b) for the dental hard tissue is chemically hardening.

In another preferred embodiment the dental filling material a) is chemically curing and the sealant b) for dental hard tissue is photo curing.

The adhesive sealant b) can further also be used as a base material, as a proximal sealant or as a fissure sealant.

Using the filling system presented here, it is possible to prepare fillings that are metal free and preferably tooth colored with a required simple, economical operative technique corresponding to the general skill of the dentist. Such filling systems are true amalgam substitutes because they not only restore the tooth's shape and function but also protect the same against caries and erosion. Analogously, such systems can also be used for root fillings.

A further aspect of the present invention is a method for filling therapy of cavities or root canals by using one of the dental filling systems described above.

The present invention shall, in the following be illustrated by the following examples.

### Example 1

The following sealants are set forth for the purpose of the ensuing creation of a filling system :
a) sealing through radical cross-linking :

| | |
|---|---|
| Bis GMA or Urethane DMA | 49.0 % per weight |
| hydroxyfunctionalized methacrylate | 29.5 % per weight |
| acid modified polymethacrylate | 20.0 % per weight |
| photoinitiator system | 1.5 % per weight |

b) sealing through low-shrinking cationic cross-linking :

| | |
|---|---|
| epoxy functionilized oligomer | 70 % per weight |
| epoxy functionilized polyol | 27 % per weight |
| cationic initiator | 3 % per weight |

c) low-shrinking composit by cationic cross-linking:

| | |
|---|---|
| epoxy functionalized resin mixture | 25 % per weight |
| x-ray active Ba/Al-borosilicate glass < 2.0 micron | 75 % per weight |

### Example 2

A cavity is sealed upon using the composition a) of example 1, and thereafter it is filled with the low-shrinking composite c) of example 1 and is finally irradiated with a polymerisation lamp for about 60 seconds. The filling thus obtained does not form any bond with the sealant having been formed upon radical cross-linking and forms therefore a dense, strainable and durable filling system due to the minimal shrinkage. There is essentially no tension transfer to the dental hard tissue.

### Example 3

Cavities, tiny lesions or fissures are sealed and cured (hardened) with a radically cross-linking sealant according to item a) of example 1). Thereafter the sealing resin according to item b) of example is applied and irradiated for about 60 seconds. The result is a stress-free sealing of said cavity, lesion or fissure. Due to the high conversion rate of the cationic cross-linking system of item b) of example 1, an inhibition-free layer is the result. Plaque, beverages like tea, coffee could not cause any discolorations. The chemical degradation does intervene only to a minor extent. The mechanical abrasion could also be diminished.

### Example 4

A cavity is filled with a composite c) of example 1). Due to the high conversion rate of the preceding cationic sealing, no chemical bond is generated between the sealant and the filling material. The result is a solid, stress-resistant filling on the one hand and a well-protected adhesive sealing of the dental hard tissue on the other hand. Due to the absence of material shrinkage-related stress, the comfort for carrying such a restoration is to be underlined.

### Reference Example 1

Conventional fillings having been placed with commercially available composites or compomers (i.e. acid-modified composites) could also be sealed-off by means of a cationic cross-linking sealant according to item b) of example 1, thus avoiding secondary caries resulting from shrinkage of the filling material.

## Claims

1. A metal free dental filling system suitable for the filling therapy of cavities or root canals, comprising
a) a dental filling material for the cavities or root canals, and
b) an adhesive, curable or self-curing sealant for the dental hard tissue.
c) wherein the adhesive sealant is suited to provide after curing an adhesive bond to dental hard tissue which is stronger than to the cured filling material, such that substantially no destructive forces are transmitted from the shrinking filling material to the adhesive sealant.

2. The dental filling of claim 1, wherein it further comprises an intermediate layer material d), which is suited for arrangement between the dental filling material and the sealant, wherein the adhesive sealant is suited to provide an adhesive bond to dental hard tissue which is stronger than to the intermediate layer material, for substantially preventing destructive forces from being transmitted from the shrinking filling material to the adhesive sealant during curing.

3. The dental filling system of claim 2, wherein the intermediate layer material is removable before the application of the filling material.

4. The dental filling system of one of the preceding claims, which is suited to provide substantially no adhesive bond between the hardened adhesive sealant and the filling material or, if present, the intermediate filling material for preventing destructive forces from being transmitted from the shrinking filling material and/or the intermediate layer material to the adhesive sealant during curing.

5. The dental filling system of one of the preceding claims, wherein the dental filling material or, if present, the intermediate layer material on the one hand as well as the adhesive sealant on the other hand are suited not show a mechanical interlocking or bond caused by homo- or co-polymerization.

6. The dental filling system of claim 5, wherein for preventing a mechanical interlocking or homo- or co-polymerization, it comprises a separation layer suitable for fully coating the sealant, preferably consisting of a painted material, such as zinc oxide-Eugenol, barrier materials, such as powders or powder coatings, glycerol-acetate-based isolating gels or primers, such as silicone-organic compounds.

7. The dental filling system of one of the claims 1 - 4, wherein the adhesive sealant, when hardened, and/or the filling material or, if present, the intermediate layer material has a low radical content and therefore is not apt for co- or homo-polymerization.

8. The dental filling system of claim 7, wherein the adhesive sealant, when hardened, and/or the filling material or, if present, the intermediate layer material are unable to undergo homo- and/or co-polymerization at the common interface surfaces.

9. The dental filling system of one of the claims 7 or 8, wherein the capacity for co-polymerization is weakened by an admixture of BisGMA bicarbonate or photo-initiators in higher concentrations or by an air blocker suitable for covering the sealant prior to light-curing for avoiding the build-up of a radical rich oxygen inhibited surface layer.

10. The dental filling system of one of the claims 1 - 4, wherein the filling material or, if present, the intermediate layer material on the one hand, as well as the hardened adhesive sealant, when hardened, on the other hand are differently polar for suppressing wetting.

11. The dental filling system of claim 10, wherein the sealant is hydrophilic or amphiphilic and the filling material is strongly hydrophobic.

12. The dental filling system of claim 1, wherein the filling material is a methacrylated substance and the adhesive sealant is suited to have a separating effect in respect to the filling material a) for suppressing a bond transmitting shrink-forces between the sealant and the filling material.

13. The dental filling system of claim 12, wherein the sealant is selected from water glass, silicone ester hydrolysates, polymerisiable silane compounds, natural resin varnishes, cyanate based varnishes, epoxide resins, epoxidated soy oils, epoxide modified silicones.

14. The dental filling system of one of the preceding claims, wherein the adhesive sealant is suitable to be used as a base material, as a proximal sealant or as a fissure sealant.

15. The dental filling system of one of the preceding claims, wherein the dental filling material and/or the sealant are photo hardening, chemically hardening, dually hardening, heat hardening or thermoplastic, or, wherein it is hardening using the principles of contact glues.

16. The dental filling system of claim 15, wherein the filling material is light curing and the sealant is chemically self-curing.

17. The dental filling system of claim 15, wherein the filling material is chemically self-curing and the sealant is light curing.

18. The dental filling material of one of the preceding claims, wherein the filling material comprises plastics, composites, ceromers, polyglasses, glass ionomer cements, carboxylate cement, phosphate cement, EBA-cement or guttapercha.

19. The dental filling system of one of the preceding claims, wherein the adhesive sealant or, if present, the intermediate layer material, or the filling material is suited to release plaque-suppressing or caries-protective agents permanently or upon request according to the principles of a smart material.

20. The dental filling system of one of the preceding claims wherein it is tooth colored.

## Patentansprüche

1. Metallfreies Zahnfüllsystem zum Füllen von Kavitäten oder Wurzelkanälen, umfassend
a) Zahnfüllmaterial für die Kavitäten oder Wurzelkanäle, und
b) Klebendes, härtendes oder selbsthärtendes Dichtungsmittel für das harte Zahngewebe,
c) Worin das klebende Dichtungsmittel eine Klebebindung mit dem harten Zahngewebe herstellen kann, die stärker ist als die Bindung mit dem Füllmaterial, so dass im Wesentlichen keine zerstörenden Kräfte vom schrumpfenden Füllmaterial auf das klebende Dichtungsmittel nach dem Aushärten übertragen werden.

2. Zahnfüllmaterial nach Anspruch 1, worin es ferner ein Zwischenschichtmaterial d) umfasst, das zwischen dem Zahnfüllmaterial und dem Dichtungsmittel angeordnet werden kann, worin das klebende Dichtungsmittel eine Klebebindung mit dem harten Zahngewebe herstellen kann, die stärker ist als die Bindung mit dem Zwischenschichtmaterial, um eine Übertragung zerstörender Kräfte vom schrumpfenden Füllmaterial auf das klebende Dichtungsmittel beim Aushärten im Wesentlichen zu vermeiden.

3. Zahnfüllsystem nach Anspruch 2, worin das Zwischenschichtmaterial vor Aufbringen des Füllmaterials entfernt werden kann.

4. Zahnfüllsystem nach einem der vorhergehenden Ansprüche, das im Wesentlichen keine Klebebindung zwischen dem ausgehärteten klebenden Dichtungsmittel und dem Füllmaterial bzw., wenn vorhanden, dem Zwischenfüllmaterial ergibt, um eine Übertragung zerstörender Kräfte vom schrumpfenden Füllmaterial und/oder dem Zwischenschichtmaterial auf das klebende Dichtungsmittel beim Aushärten im Wesentlichen zu vermeiden.

5. Zahnfüllsystem nach einem der vorhergehenden Ansprüche, worin das Zahnfüllmaterial oder, wenn vorhanden, das Zwischenschichtmaterial einerseits und das klebende Dichtungsmittel andererseits keine mechanische Arretierung oder Bindung durch Homo- oder Copolymerisation eingehen.

6. Zahnfüllsystem nach Anspruch 5, worin es zur Vermeidung einer mechanischen Arretierung oder Homo- oder Copolymerisation eine Trennschicht umfasst, die das Dichtungsmittel vollständig überziehen kann und die vorzugsweise aus einem lackierten Material, beispielsweise aus Zinkoxid-Eugenol, Barrierematerialien, wie z.B. Pulver oder Pulverbeschichtungen, Isoliergele auf Glycerolacetat-Basis oder Grundiermittel, wie z.B. organische Silikonverbindungen besteht.

7. Zahnfüllsystem nach einem der Ansprüche 1-4, worin das klebende Dichtungsmittel nach dem Aushärten und/oder das Füllmaterial oder, wenn vorhanden, das Zwischenschichtmaterial einen geringen Gehalt an freien Radikalen aufweisen und deshalb nicht zur Co- oder Homopolymerisation neigen.

8. Zahnfüllsystem nach Anspruch 7, worin das klebende Dichtungsmittel nach dem Aushärten und/oder das Füllmaterial oder, wenn vorhanden, das Zwischenschichtmaterial keine Homo- und/oder Copolymerisation an den gemeinsamen Schnittstellen eingehen können.

9. Zahnfüllsystem nach einem der Ansprüche 7 oder 8, worin die Fähigkeit zur Copolymerisation durch Beimischung von BisGMA-Bicarbonat oder Photoinitiatoren in höheren Konzentrationen oder durch einen Luftblocker, der das Dichtungsmittel vor der Lichthärtung abdecken kann, abgeschwächt wird, um den Aufbau einer radikalreichen Sauerstoff-inhibierten Oberflächenschicht zu vermeiden.

10. Zahnfüllsystem nach einem der Ansprüche 1-4, worin das Füllmaterial oder, wenn vorhanden, das Zwischenschichtmaterial einerseits und das ausgehärtete klebende Dichtungsmittel nach dem Aushärten andererseits zur Unterdrückung der Benetzung unterschiedliche Polarität aufweisen.

11. Zahnfüllsystem nach Anspruch 10, worin das Dichtungsmittel hydrophil oder amphiphil und das Füllmaterial stark hydrophob ist.

12. Zahnfüllsystem nach Anspruch 1, worin das Füllmaterial eine methacrylierte Substanz ist und das Dichtungsmittel einen Trenneffekt gegenüber dem Füllmaterial a) ausüben kann, um eine Schrumpfkräfte übertragende Bindung zwischen dem Dichtungsmittel und dem Füllmaterial zu unterdrücken.

13. Zahnfüllsystem nach Anspruch 12, worin das Dichtungsmittel unter Wasserglas, Silikonester-Hydrolysaten, polymerisierbaren Silanverbindungen, Naturharz-Lacken, Lacken auf Cyanatbasis, Epoxyharzen, epoxidierten Sojaölen und mit Epoxid modifizierten Silikonen ausgewählt wird.

14. Zahnfüllsystem nach einem der vorhergehenden Ansprüche, worin das klebende Dichtungsmittel als Basismaterial, als proximales Dichtungsmittel oder als Rissdichtungsmittel verwendet werden kann.

15. Zahnfüllsystem nach einem der vorhergehenden Ansprüche, worin das Zahnfüllmaterial und/oder das Dichtungsmittel lichthärtend, chemisch härtend, doppelthärtend, wärmehärtend oder thermoplastisch sind, oder worin es nach den Prinzipien von Kontaktklebstoffen aushärten kann.

16. Zahnfüllsystem nach Anspruch 15, worin das Füllmaterial lichthärtend ist und das Dichtungsmittel chemisch selbsthärtend ist.

17. Zahnfüllsystem nach Anspruch 15, worin das Füllmaterial chemisch selbsthärtend und das Dichtungsmittel lichthärtend ist.

18. Zahnfüllmaterial nach einem der vorhergehenden Ansprüche, worin das Füllmaterial Kunststoffe, Verbundmaterialien, Ceromere, Polygläser, Glas-Ionomer-Zemente, carboxylierten Zement, Phosphatzement, EBA-Zement oder Guttapercha umfasst.

19. Zahnfüllmaterial nach einem der vorhergehenden Ansprüche, worin das klebende Dichtungsmittel oder, wenn vorhanden, das Zwischenschichtmaterial, oder das Füllmaterial ständig oder bei Bedarf Plaque-unterdrückende oder vor Karies schützende Mittel nach den Prinzipien eines smarten Materials freisetzen kann.

20. Zahnfüllmaterial nach einem der vorhergehenden Ansprüche, das zahnfarben ist.

## Revendications

1. Système d'obturation dentaire sans métal approprié pour un traitement d'obturation de caries ou de canaux radiculaires, comprenant
a) un matériau d'obturation dentaire pour caries ou canaux radiculaires, et
b) un agent de scellement adhésif, durcissable ou autopolymérisable pour le tissu dur dentaire,
c) dans lequel l'agent de scellement adhésif convient pour fournir une liaison adhésive au tissu dur dentaire qui est plus forte que la liaison au matériau d'obturation durci, de telle sorte que pratiquement aucune force destructive ne soit transmise du matériau d'obturation en train de se rétracter à l'agent de scellement adhésif après le durcissement.

2. Système d'obturation dentaire de la revendication 1, qui comprend en outre un matériau de couche intermédiaire d), qui convient pour être placé entre le matériau d'obturation dentaire et l'agent de scellement, dans lequel l'agent de scellement adhésif convient pour fournir une liaison adhésive au tissu dur dentaire qui est plus forte que la liaison au matériau de couche intermédiaire, pour empêcher essentiellement les forces destructives d'être transmises, durant le durcissement, du matériau d'obturation en train de se rétracter à l'agent de scellement adhésif.

3. Système d'obturation dentaire de la revendication 2, dans lequel le matériau de couche intermédiaire peut être retiré avant l'application du matériau d'obturation.

4. Système d'obturation dentaire de l'une des revendications précédentes, qui convient pour fournir une force adhésive essentiellement nulle entre l'agent de scellement adhésif durci et le matériau d'obturation ou, si il est présent, le matériau d'obturation intermédiaire pour empêcher les forces destructives d'être transmises du matériau d'obturation en train de se rétracter et/ou du matériau de couche intermédiaire à l'agent de scellement adhésif durant le durcissement.

5. Système d'obturation dentaire de l'une des revendications précédentes, dans lequel le matériau d'obturation dentaire ou, s'il est présent, le matériau de couche intermédiaire d'une part, ainsi que le matériau de scellement adhésif d'autre part, conviennent pour ne pas présenter de verrouillage mécanique ou de liaison provoquée par une homopolymérisation ou une copolymérisation.

6. Système d'obturation dentaire de la revendication 5, qui, pour empêcher un verrouillage mécanique ou une homopolymérisation ou copolymérisation, comprend une couche de séparation appropriée pour revêtir entièrement l'agent de scellement, constituée de préférence d'un matériau peint, tel que de l'oxyde de zinc-Eugénol, de matériaux de barrière, tels que des poudres ou des revêtements en poudre, de gels isolants à base d'acétate de glycérol ou d'apprêts, tels que des composés organiques de silicone.

7. Système d'obturation dentaire de l'une des revendications 1-4, dans lequel l'agent de scellement adhésif, lorsqu'il est durci, et/ou le matériau d'obturation ou, s'il est présent, le matériau de couche intermédiaire, possède une faible teneur en radicaux et par conséquent n'est pas susceptible de copolymérisation ou d'homopolymérisation.

8. Système d'obturation dentaire de la revendication 7, dans lequel l'agent de scellement adhésif, lorsqu'il est durci, et/ou le matériau d'obturation ou, s'il est présent, le matériau de couche intermédiaire, sont incapables de subir une homopolymérisation et/ou une copolymérisation au niveau des surfaces des interfaces communes.

9. Système d'obturation dentaire de l'une des revendications 7 ou 8, dans lequel l'aptitude à la copolymérisation est affaiblie par un ajout de bicarbonate de BisGMA ou de photo-initiateurs en plus fortes concentrations, ou par un produit d'étanchéité approprié pour recouvrir l'agent de scellement avant le durcissement à la lumière pour éviter l'accumulation d'une couche superficielle inhibée par l'oxygène, riche en radicaux.

10. Système d'obturation dentaire de l'une des revendications 1 - 4, dans lequel le matériau d'obturation ou, s'il est présent, le matériau de couche intermédiaire d'une part, ainsi que l'agent de scellement adhésif durci, lorsqu'il est durci, d'autre part, ont des polarités différentes pour empêcher le mouillage.

11. Système d'obturation dentaire de la revendication 10, dans lequel l'agent de scellement est hydrophile ou amphiphile et le matériau d'obturation est fortement hydrophobe.

12. Système d'obturation dentaire de la revendication 1, dans lequel le matériau d'obturation est une substance méthacrylée et l'agent de scellement adhésif convient pour avoir un effet de séparation par rapport au matériau d'obturation a) pour empêcher une liaison transmettant des forces de rétraction entre l'agent de scellement et le matériau d'obturation.

13. Système d'obturation dentaire de la revendication 12, dans lequel l'agent de scellement est choisi parmi le verre d'eau, les hydrolysats d'ester de silicone, les composés de silane polymérisables, les vernis de résines naturelles, les vernis à base de cyanate, les résines époxyde, les huiles de soja époxydées, les silicones modifiées par un époxyde.

14. Système d'obturation dentaire de l'une des revendications précédentes, dans lequel l'agent de scellement adhésif convient pour être utilisé comme matériau de base, comme agent de scellement proximal ou comme agent de scellement de fissures.

15. Système d'obturation dentaire de l'une des revendications précédentes, dans lequel le matériau d'obturation dentaire et/ou l'agent de scellement sont photodurcissables, durcissables chimiquement, durcissables par les deux techniques, thermodurcissables ou thermoplastiques, ou dans lequel il est durci au moyen des principes des colles de contact.

16. Système d'obturation dentaire de la revendication 15, dans lequel le matériau d'obturation est durcissable à la lumière et l'agent de scellement est autopolymérisable par voie chimique.

17. Système d'obturation dentaire de la revendication 15, dans lequel le matériau d'obturation est autopolymérisable par voie chimique et l'agent de scellement est durcissable à la lumière.

18. Système d'obturation dentaire de l'une des revendications précédentes, dans lequel le matériau d'obturation comprend des matières plastiques, des composites, des céromères, des polyverres, des ciments au verre ionomère, du ciment au carboxylate, du ciment au phosphate, du ciment EBA ou de la gutta-percha.

19. Système d'obturation dentaire de l'une des revendications précédentes, dans lequel l'agent de scellement adhésif ou, s'il est présent, le matériau de couche intermédiaire, ou le matériau d'obturation, convient pour éliminer, de façon permanente ou à la demande, des agents empêchant la formation de plaque dentaire ou des agents de protection contre les caries sur le principe d'un matériau intelligent.

20. Système d'obturation dentaire de l'une des revendications précédentes, qui est de la couleur des dents.
